# DEMANDE DE BREVET EUROPEEN

(11) **EP 3 761 441 A1**
(43) Date de publication de la demande: **06.01.2021**
(21) Numéro de dépôt: 19184151.9
(22) Date de dépôt: 03.07.2019
(51) Int. Cl.: H01P 1/203, A61B 5/145, H01P 7/08, A61B 5/1477, A61B 5/05

(54) **DISPOSITIF DE MESURE DE LA GLYCÉMIE SANS CONTACT**

(71) Demandeur: Marggraf, 88000 Epinal (FR); Université de Lorraine, 54000 Nancy (FR)
(72) Inventeur: BOMBARDE, Rémi, 54600 Villers-les-Nancy (FR); ROUANE, Amar, 54180 Houdemont (FR); ZIDANE, Mohamed-Amine, 54000 Nancy (FR); HAMOUDA, Chérif, 75019 Paris (FR)
(74) Mandataire: Office Freylinger

(57) **Abrégé**

Un dispositif de mesure de la glycémie dans un corps vivant comporte une antenne (1) destinée à être placée à proximité du corps vivant, une unité électronique (2) pour alimenter l'antenne (1) et mesurer un signal de retour de l'antenne (1). L'antenne (1, 1') comporte :
- une ligne de transmission (10, 10') s'étendant sur une première face (11) ou une deuxième face (12) d'un substrat diélectrique plan et connectée à l'unité électronique (2) ;
- un plan de masse (13) s'étendant sur au moins une partie de la deuxième face (12) du substrat ;
- au moins un résonateur (14) s'étendant sur la première face (11) à proximité de la ligne de transmission (10, 10') ;
l'unité électronique (2) étant prévue pour fournir à l'antenne (1) un signal à une fréquence prédéterminée comprise entre 0,9 et 1,6 GHz, mesurer un signal de retour, déterminer les caractéristiques électriques de l'antenne (1) et en déduire un taux de glycémie dans le corps vivant.

## Description

### Domaine

L'invention concerne un dispositif de mesure de la glycémie dans un corps vivant, en particulier dans le corps humain, non invasive, fondée sur l'interaction d'un signal à hyper-fréquence avec la molécule de glucose.

### Technique antérieure

Certaines personnes atteintes d'un dysfonctionnement de la régulation de la glycémie, c'est-à-dire de la concentration en glucose dans le sang, doivent effectuer plusieurs fois par jour une mesure de la glycémie afin d'ajuster un traitement correctif. La technique la plus répandue consiste à prélever un peu de sang avec une lancette et à analyser ce prélèvement à l'aide d'une bandelette réactive et d'un lecteur de glycémie. Ceci entraîne un inconfort d'utilisation et un risque d'infection du fait que la peau doit être percée à chaque prélèvement.

Des recherches ont été menées pour utiliser un signal à hyper-fréquence et de détecter l'influence de la concentration en glucose sur ledit signal. Le document KR 10-1184420 B1 montre la réalisation d'une antenne avec un substrat plan, sur une face duquel une bande conductrice s'étend en spirale. Un contenant avec une solution de glucose est déposée sur le substrat au niveau de la spirale et le coefficient de réflexion à l'entrée mesuré par une électronique appropriée. Le document montre une sensibilité de ce coefficient à la concentration en glucose dans la solution quand le signal d'excitation est à la fréquence de 7,7 GHz.

Ce dispositif a l'inconvénient de travailler à une fréquence très élevée, ce qui implique des coûts de fabrication élevés du fait que les techniques de fabrications de l'antenne et de l'électronique de mesure doivent être très précises. De plus, plus la fréquence du signal est élevée, plus la taille de l'antenne est petite. Pour l'utilisateur, ceci implique d'être précis dans le positionnement de l'antenne lors de la mesure.

### Exposé de l'invention

L'invention vise à fournir un dispositif de mesure de la glycémie qui permet un coût de fabrication raisonnable et une utilisation aisée. À cette fin, l'invention a pour objet un dispositif de mesure de la glycémie dans un corps vivant comportant une antenne destinée à être placée à proximité du corps vivant, une unité électronique pour alimenter l'antenne et mesurer un signal de retour de l'antenne, caractérisé en ce que l'antenne comporte : une ligne de transmission s'étendant sur une première face ou une deuxième face d'un substrat diélectrique plan et connecté à l'unité électronique ; un plan de masse s'étendant sur au moins une partie de la deuxième face du substrat ; au moins un résonateur s'étendant sur la première face à proximité de la ligne de transmission ; l'unité électronique étant prévue pour fournir à l'antenne un signal à une fréquence prédéterminée comprise entre 0,9 et 1,6 GHz, mesurer un signal de retour, déterminer les caractéristiques électriques de l'antenne et en déduire un taux de glycémie dans le corps vivant.

On a constaté que cette forme d'antenne permet d'utiliser un signal dont la fréquence est plus basse que dans l'art antérieur et avec une excellente sensibilité. La ligne de transmission sert d'excitateur qui met le résonateur en résonance électrique. Il sert également de capteur en étant influencé par le signal de retour du résonateur. Les caractéristiques de l'antenne sont déterminées pour être en résonance avec la fréquence prédéterminée. Lorsqu'une solution contenant du glucose est placée au-dessus du plan de l'antenne et centrée sur le résonateur, les caractéristiques de l'antenne, et donc le signal de sortie sont influencées par le taux de glucose dans la solution, la variation de la concentration du glucose dans le milieu impliquant une variation de la permittivité de ce milieu. Ainsi, on peut accéder directement à une mesure du taux de glycémie dans le sang d'un corps vivant sans procédé invasif. Le confort d'une personne qui l'utilise en est amélioré. De plus, il est possible ainsi de mesurer beaucoup plus fréquemment le taux de glycémie et de le suivre quasiment en temps réel si besoin. Par rapport à une mesure avec une antenne à 7,7 GHz, l'antenne construite ici a une plus grande taille, ce qui permet une moindre sensibilité au positionnement de l'antenne sur le corps vivant pour effectuer la mesure. L'utilisation en est facilitée. De plus, les techniques de fabrication nécessitent moins de précision, ce qui permet d'abaisser le coût de fabrication à la fois de l'antenne et de l'unité électronique pour effectuer la mesure.

Selon un mode de réalisation, l'antenne comporte un nombre pair de résonateurs disposés de part et d'autre de la ligne de transmission. On augmente la surface d'émission et donc de mesure sans modifier la fréquence de résonance.

Selon des dispositions constructives, le résonateur est choisi dans un groupe comprenant un résonateur à anneau divisé simple, un résonateur à anneau divisé double.

Selon des dispositions constructives, les anneaux sont elliptiques, circulaires, polygonaux, rectangulaires, carrés ou triangulaires. Ces dispositions permettent de se conformer à différentes contraintes d'encombrement.

Selon un mode de réalisation, l'antenne comporte quatre résonateurs à doubles anneaux circulaires divisés.

Selon un mode de réalisation, la ligne de transmission est un microruban s'étendant sur la première face, le plan de masse s'étendant sur toute la deuxième face. Les résonateurs sont donc sur la même face que le microruban de part et d'autre de celui-ci.

Selon un autre mode de réalisation, la ligne de transmission est une ligne coplanaire s'étendant sur la deuxième face et séparant le plan de masse en deux demi-plans par des intervalles sensiblement constants.

Selon un mode de réalisation, l'unité électronique mesure un coefficient de réflexion à l'entrée S11, un coefficient de transmission direct S21 ou un déphasage entre le signal d'entrée et le signal réfléchi par l'antenne, et déduit le taux de glycémie de l'une de ces mesures ou d'une combinaison d'au moins deux de celles-ci.

Selon un perfectionnement, l'unité électronique neutralise la mesure si le déphasage est inférieur à un seuil prédéterminé. On a constaté que le déphasage entre le signal d'entrée et le signal de sortie était peu important lorsqu'aucun échantillon n'était en regard de l'antenne, mais que celui-ci était sensiblement influencé par la présence d'un échantillon de mesure contenant du glucose, ce qui permet de discriminer les situations avec ou sans échantillon à mesurer.

Le seuil prédéterminé est compris par exemple entre 30 et 40°.

### Brève description des figures

L'invention sera mieux comprise et d'autres particularités et avantages apparaîtront à la lecture de la description qui va suivre, la description faisant référence aux dessins annexés parmi lesquels :
- la figure 1 est une vue schématique fonctionnelle d'un dispositif conforme à un mode de réalisation de l'invention ;
- la figure 2 est une vue de dessus de l'antenne du dispositif de la figure 1 ;
- la figure 3 est une vue de dessous de l'antenne de la figure 2 ;
- la figure 4 est un diagramme montrant une amplitude liée au du signal S11 mesuré par l'électronique en fonction de la glycémie d'échantillons de sang ;
- la figure 5 est un diagramme montrant le déphasage mesuré par l'électronique en fonction de la glycémie d'échantillons de sang ;
- la figure 6 est un diagramme montrant le déphasage mesuré par l'électronique pour cinq cas.
- la figure 7 est une vue de dessus de l'antenne du dispositif selon un deuxième mode de réalisation ;
- la figure 8 est une vue de dessous de l'antenne de la figure 7.

### Description détaillée

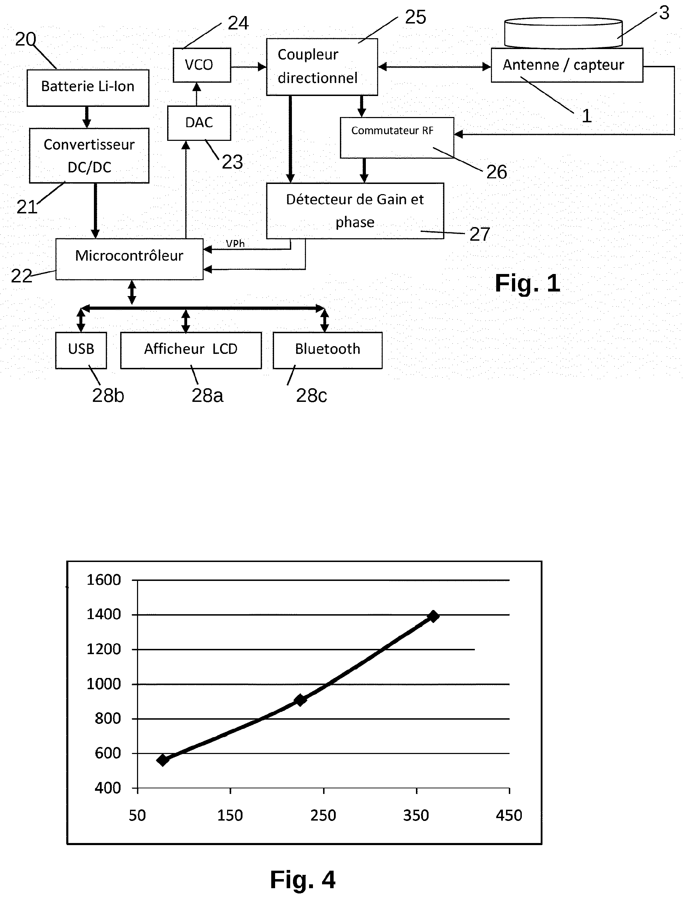

Selon un premier mode de réalisation de l'invention un dispositif de mesure de la glycémie dans un corps vivant est représenté de manière schématique sur la figure 1. Le dispositif comporte une antenne 1 destinée à être placée à proximité du corps vivant et une unité électronique 2 pour alimenter l'antenne 1 et mesurer un signal de retour de l'antenne 1.

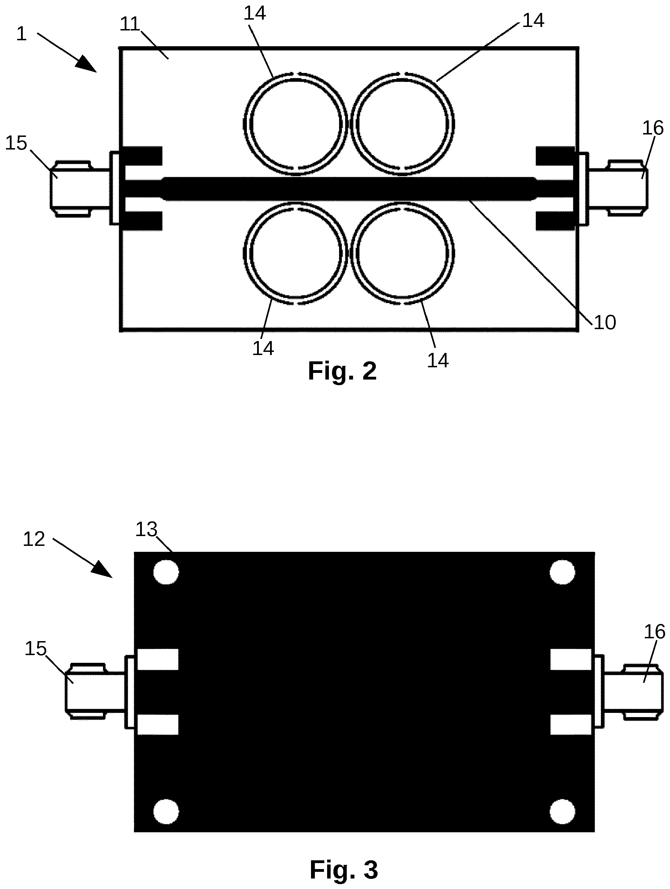

L'antenne 1, telles que représentée sur les figures 2 et 3, est réalisée à partir d'un substrat diélectrique en forme de plaque, par exemple à base de tissu de verre enrobé dans une matrice de polyépoxyde, communément utilisé dans l'industrie électronique et connu sous la référence FR4. L'antenne 1 comporte :
- un microruban 10 s'étendant sur une première face 11 du substrat et connecté à l'unité électronique 2 ; le microruban 10 est appelé « microstrip » en anglais et est une piste de cuivre ;
- un plan de masse 13 s'étendant sur une deuxième face 12 du substrat, formé par une couche de cuivre ;
- quatre résonateurs 14 à anneau divisé double s'étendant sur la première face 11 de part et d'autre du microruban 10 par paires symétriques. Les résonateurs 14 sont également appelés « SRR » pour « split ring resonator » en anglais.

Les dimensions et les caractéristiques de l'antenne 1 sont déterminées pour que la fréquence de résonance soit incluse dans l'intervalle de 0,9 à 1,6 GHz.

L'unité électronique 2 est prévue pour fournir à l'antenne 1 un signal à une fréquence prédéterminée proche de la fréquence de résonance, mesurer un signal de retour, déterminer les caractéristiques électriques de l'antenne 1 et en déduire un taux de glycémie dans le corps vivant. Pour cela, l'unité électronique 2 comporte une batterie 20 alimentant un microprocesseur 22 et ses accessoires par l'intermédiaire d'un convertisseur 21. Le microprocesseur 22 pilote un convertisseur numérique-analogique 23 lequel contrôle un oscillateur 24 commandé en tension pour générer un signal sinusoïdal. Le signal sinusoïdal est fourni à un coupleur directionnel 25 dont la sortie principale est dirigée vers l'antenne 1, et plus précisément à une extrémité du microruban 10 muni d'un connecteur d'entrée 15. L'autre extrémité du microruban 10 est munie d'un connecteur de sortie 16 qui est relié à un commutateur 26 à radiofréquence qui sélectionne soit un signal de sortie fourni par le connecteur de sortie 16, soit le signal issu d'une ligne de mesure du coupleur directionnel 25. Le signal sélectionné est envoyé vers un détecteur de gain et de phase 27 qui fournit deux signaux, un signal de phase Vph et un signal d'amplitude Vma au microprocesseur 22. Le microprocesseur 22 numérise ces deux signaux et les traite pour déterminer un déphasage ϕ et les coefficients de réflexion à l'entrée S11 et de transmission direct S21. À partir de ces mesures, un taux de glycémie est calculé. Le taux de glycémie peut alors être par exemple affiché sur un écran 28a, mis à disposition via une interface numérique telle qu'un port USB 28b ou une interface Bluetooth 28c. Le taux de glycémie est par exemple une fonction directe du coefficient de réflexion à l'entrée S11, par exemple linéaire ou par une loi prédéterminée expérimentale. De la même manière, on peut utiliser également le coefficient de transmission direct S21 seul. On peut également utiliser une combinaison de ces deux coefficients. Le déphasage ϕ est utilisé pour déterminer si la mesure est faite en présence d'un corps à mesurer. Si la valeur est inférieure à un seuil prédéterminé, par exemple compris entre 30 et 40°, on considère qu'aucun corps vivant n'est dans le champ de l'antenne 1 et on invalide toute mesure.

La variation de la concentration du glucose dans le milieu implique une variation de la permittivité de ce milieu. Par conséquent, l'impédance de ce dernier varie, on peut exploiter cette dernière propriété pour extraire les informations sur la glycémie, en utilisant les paramètres S. Dans le mode de réalisation exposé, on envoie une onde incidente d'un premier port, le connecteur d'entrée 15, vers un second, le connecteur de sortie 16. Une partie de l'onde incidente sera réfléchie vers le port 15 et une autre partie sera transmise vers le second port 16. Le paramètre S11 représente la partie réfléchie de la puissance incidente et le paramètre S21 représente la puissance transmise vers le second port 16. Le principe de la méthode utilisée consiste à trouver une corrélation entre la variation de la concentration du glucose et les paramètres S. La puissance absorbée par le résonateur dépend de la permittivité du milieu vu que la capacité équivalente du résonateur dépend du milieu.

On a conduit des essais de mesure avec ce dispositif. On a versé 6,5 ml de sang dans un coupelle 3 en verre avec des concentrations en glucose variables. On a placé la coupelle sur l'antenne 1 puis on a effectué les mesures. Les résultats sont présentés sur les figures 4 à 6. Dans tous ces cas, le taux de glycémie a été déterminé par une mesure avec un glucomètre à bandelette.

La figure 4 est un diagramme avec en ordonnée une amplitude liée au signal S11 mesuré par l'électronique et en abscisse la glycémie des échantillons de sang. On constate que la valeur absolue du signal S11 augmente de manière sensible avec le taux de glycémie.

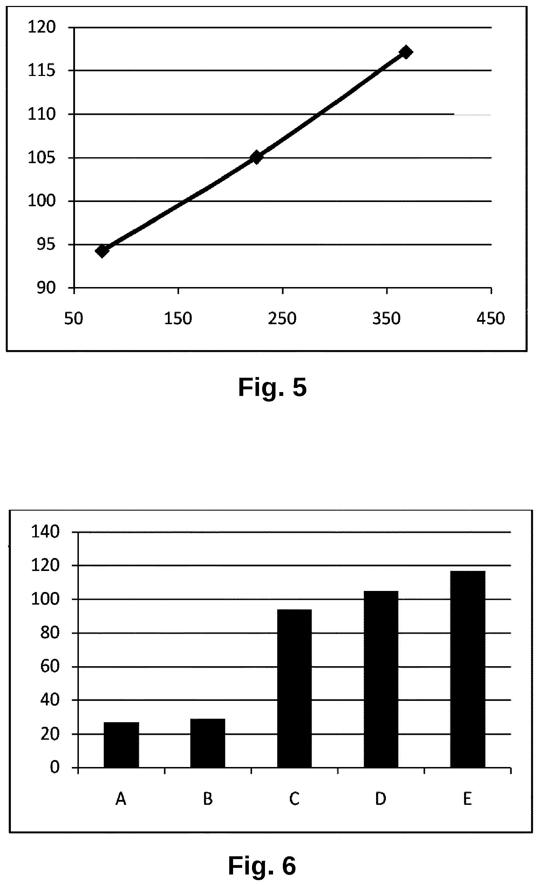

La figure 5 est un diagramme montrant le déphasage ϕ en degrés mesuré par l'électronique en fonction de la glycémie d'échantillons de sang pour les échantillons représentés également sur la figure 4. On constate ici aussi une augmentation du déphasage ϕ en fonction du taux de glycémie.

La figure 6 est un graphe à barres représentant le déphasage ϕ pour les échantillons de la figure 4 et pour deux cas supplémentaires. Les cinq cas sont les suivants :

| **Cas** | **Description** |
|---|---|
| A | Mesure sans coupelle |
| B | Mesure avec coupelle vide |
| C | Coupelle avec sang à glycémie de 77 mg/dl |
| D | Coupelle avec sang à glycémie de 225 mg/dl |
| E | Coupelle avec sang à glycémie de 368 mg/dl |

La figure 6 montre très clairement que les cas avec un déphasage faible sont des mesures sans présence de corps à mesurer (cas A et B), et est donc un critère pour éliminer ces mesures.

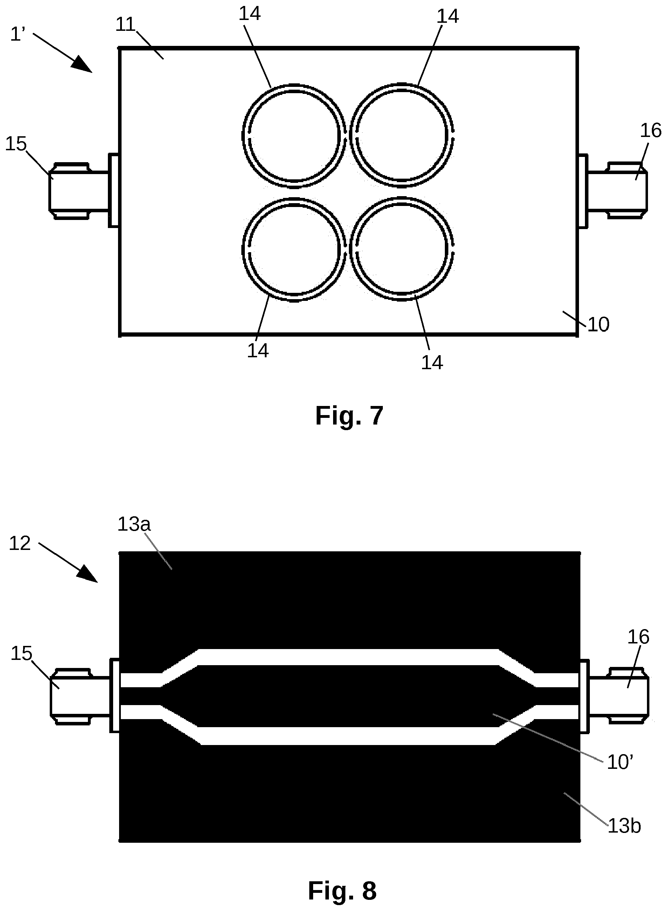

Dans un deuxième mode de réalisation, montré sur les figures 7 et 8, l'antenne l' comporte une ligne coplanaire 10' à la place du microruban. La ligne coplanaire 10' est une piste conductrice rectiligne sur la deuxième face 12 et qui sépare en deux demi-plans 13a, 13b le plan de masse. De ce fait, les deux demis-plans 13a, 13b sont séparés de la ligne coplanaire 10' par des intervalles sensiblement constants. Les résonateurs 14 restent identiques au premier mode de réalisation sur la première face 11. Les connecteurs 15, 16 sont reliés aux deux extrémités de la ligne coplanaire 10'. Le mode de fonctionnement du dispositif reste identique au premier mode de réalisation avec cette antenne 1'.

L'invention n'est pas limitée au mode de réalisation décrit uniquement à titre d'exemple. Les résonateurs pourraient être elliptiques, circulaires, polygonaux, rectangulaires, carrés ou triangulaires. Ce pourrait être aussi des résonateurs à anneau divisé simple ou à anneau fermé. Les formes peuvent également être des formes complémentaires, c'est-à-dire que les anneaux sont des parties non conductrices découpées dans une surface conductrice.

## Revendications

1. Dispositif de mesure de la glycémie dans un corps vivant comportant une antenne (1) destinée à être placée à proximité du corps vivant, une unité électronique (2) pour alimenter l'antenne (1) et mesurer un signal de retour de l'antenne (1, 1'), **caractérisé en ce que** l'antenne (1, 1') comporte :
- une ligne de transmission (10, 10') s'étendant sur une première face (11) ou une deuxième face (12) d'un substrat diélectrique plan et connectée à l'unité électronique (2) ;
- un plan de masse (13) s'étendant sur au moins une partie de la deuxième face (12) du substrat ;
- au moins un résonateur (14) s'étendant sur la première face (11) à proximité de la ligne de transmission (10, 10') ;
l'unité électronique (2) étant prévue pour fournir à l'antenne (1,1 ') un signal à une fréquence prédéterminée comprise entre 0,9 et 1,6 GHz, mesurer un signal de retour, déterminer les caractéristiques électriques de l'antenne (1) et en déduire un taux de glycémie dans le corps vivant.

2. Dispositif selon la revendication 1, dans lequel l'antenne (1) comporte un nombre pair de résonateurs (14) disposés de part et d'autre de la ligne de transmission (10).

3. Dispositif selon l'une des revendications 1 ou 2, dans lequel le résonateur (14) est choisi dans un groupe comprenant un résonateur à anneau divisé simple, un résonateur à anneau divisé double.

4. Dispositif selon la revendication 3, dans lequel les anneaux sont elliptiques, circulaires, polygonaux, rectangulaires, carrés ou triangulaires.

5. Dispositif selon la revendication 3, dans lequel l'antenne (1) comporte quatre résonateurs (14) à doubles anneaux circulaires divisés.

6. Dispositif selon l'une des revendications précédentes, dans lequel la ligne de transmission est un microruban (10) s'étendant sur la première face (11), le plan de masse (13) s'étendant sur toute la deuxième face (12).

7. Dispositif selon l'une des revendications 1 à 5, dans lequel la ligne de transmission est une ligne coplanaire (10') s'étendant sur la deuxième face (12) et séparant le plan de masse en deux demi-plans (13a, 13b) par des intervalles sensiblement constants.

8. Dispositif selon l'une des revendications précédentes, dans lequel l'unité électronique (2) mesure un coefficient de réflexion à l'entrée S11, un coefficient de transmission direct S21 ou un déphasage (ϕ) entre le signal d'entrée et le signal réfléchi par l'antenne (1), et déduit le taux de glycémie de l'une de ces mesures ou d'une combinaison d'au moins deux de celles-ci.

9. Dispositif selon la revendication 8, dans lequel l'unité électronique (2) neutralise la mesure si le déphasage (ϕ) est inférieur à un seuil prédéterminé.

10. Dispositif selon la revendication 9, dans le seuil prédéterminé est compris entre 30 et 40°.
